# EUROPÄISCHE PATENTSCHRIFT

(11) **EP 1 947 973 B1**
(45) Veröffentlichungstag und Bekanntmachung des Hinweises auf die Patenterteilung: **09.04.2014**
(21) Anmeldenummer: 06818586.7
(22) Anmeldetag: 16.11.2006
(51) Int. Cl.: A61F 5/37

(54) **EINSTELLGURT**
ADJUSTABLE BELT
CEINTURE RÉGLABLE

(30) Priorität: 18.11.2005 DE 202005018181 U; 18.11.2005 DE 202005018183 U
(43) Veröffentlichungstag der Anmeldung: 30.07.2008
(73) Patentinhaber: Sanchez, Alexander, 21266 Jesteburg (DE)
(72) Erfinder: Sanchez, Alexander, 21266 Jesteburg (DE)
(74) Vertreter: Richter Werdermann Gerbaulet Hofmann
(86) Internationale Anmeldenummer: PCT/EP2006/010998
(87) Internationale Veröffentlichungsnummer: WO 2007/057183

(56) Entgegenhaltungen:
- DE-A1- 1 942 535
- GB-A- 2 219 742
- US-A- 2 783 516
- US-A- 2 884 675
- US-A- 2 916 742

## Beschreibung

### Technisches Gebiet

Die Erfindung betrifft einen Gurt, vorzugsweise aus Textilwaren oder Lederwaren, mit Ösen.

### Stand der Technik

Ein solcher Gurt ist aus der Druckschrift US 2 884 675 A bekannt.

Die US 2 884 675 A offenbart dabei eine besondere konstruktive Ausgestaltung einer Schnalle eines Gürtels oder Gurtes.

Ein derartiger Gurt kann u. U. vielerlei Verwendung finden, beispielsweise in Form eines Leibgurtes im medizinischen Bereich und im Pflegebereich. Dabei handelt es sich um Leibgurte, die den Körper einer Person im Bauchbereich umschließen und ebenfalls mit Verschlussmitteln versehen sind, um den Bauchbereich der Person an einer Sitzgelegenheit zu binden. Die Personen sind vornehmlich körperbehinderte oder gehunfähige Personen, die zumindest für eine gewisse Zeit im Rollstuhl verbleiben müssen. Auch kann es sich bei den Personen im Rollstuhl um Patienten handeln, die eine Gefährdung für das Pflegepersonal oder für ihre Mitmenschen darstellen, sobald sie sich von dem Leibgurt lösen können.

Bei den Verschlussmitteln für derartige Leibgurte können Magnetschlösser verwendet werden, bei denen ein Steckschaft durch die Ösen geführt wird und in die Einschieböffnung des Gehäuses gesteckt und sodann verriegelt wird. Bei dem aus der DE-PS 1942535 bekannten Magnetschloss dienen dazu dem Gehäuse zwei spiegelbildlich angeordnete Riegelelemente, die kippbeweglich um einen jeweiligen Auflagepunkt angeordnet sind, wobei sie durch eine Feder in eine Verriegelungsposition vorgespannt werden. In dieser Verriegelungsposition greifen sie in die Sperrrille eines in das Gehäuse eingesteckten Sockelschaftes eines Sockelteils ein. Durch einen speziellen Schlüssel oder durch einen von außen aufgesetzten Magneten können die Riegelelemente dann gegen die Feder gekippt werden, wodurch sie aus der Sperrrille des Sockelteiles ausrasten und somit eine Trennung von Gehäuseteil und Sockelteil ermöglichen. Patienten bzw. Mitpatienten können ein solches Magnetschloss nicht öffnen. Allerdings muss hierbei jeder einzelne Einstellpunkt mit einer Öse erreicht werden. Da dazu das eine Gurtende zum Spannen durch eine Metallschlaufe gezogen und umgeschlagen werden muss, wären beispielsweise bei bekannten Leibgurten für einen Einstellbereich von 45 cm entsprechend 90 cm geöster Gurt erforderlich, was eine Ösenzahl von 45 bedeuten würde. Schon dieses spezielle Beispiel verdeutlicht, dass eine solch hohe Anzahl von Ösen schon aus rein konstruktionstechnischen Gründen nicht wünschenswert ist und auch unter dem Gesichtspunkt des Materialeinsatzes nicht erstrebenswert sein kann.

### Darstellung der Erfindung, Aufhabe, Lösung, Vorteile

Es ist daher Aufgabe der Erfindung, einen Gurt der eingangs genannten Art zur Verfügung zu stellen, der eine möglichst geringe Anzahl von Ösen aufweist, ohne den Einstellbereich des Gurtes einzuschränken. Auch soll mit möglichst wenig Material ein großer Einstellbereich erreicht werden.

Diese Aufgabe wird durch einen Gurt mit den in Anspruch 1 bzw. 8 angegebenen Merkmalen gelöst.

Dadurch, dass an den Ösen ein Verschlussmittel anbringbar ist und der Gurt mehrere Schlaufen aufweisen kann, lässt sich gewissermaßen das Prinzip einer Gangschaltung realisieren. So ist es beispielsweise möglich, mit Hilfe von zwei Schlaufen (statt nur einer) und drei Ösen (statt nur einer) für die Anbringung des Verschlussmittels sechs Einstellbereiche gemäß dem Produkt 2 x 3 einzustellen. Denkbar ist jedoch auch der umgekehrte Weg, vergleichbar mit einer umgekehrten Anordnung von Zahnrädern, indem eine 1 Öse über die beiden Metallschlaufen die beiden ersten Einstellbereiche, eine 2 Öse über die beiden Metallschlaufen die beiden zweiten Einstellbereiche und schließlich eine 3 Öse über die beiden Metallschlaufen die beiden dritten Einstellbereiche verfügbar machen, also ebenfalls insgesamt sechs Einstellbereiche.

Es ist daher auch vorgesehen, dass der Gurt zwei Schlaufen aufweist und das Verschlussmittel für drei Ösen vorgesehen ist.

Durch weitere Ösen und Schlaufen lassen sich weitere Einstellbereiche realisieren. Es ist somit von Vorteil, dass der Gurt drei Schlaufen aufweist und das Verschlussmittel für vier Ösen vorgesehen ist.

Die Anordnung der Ösen und Schlaufen muss dabei derart erfolgen, dass die kleinen Einstellbereiche einen gesamten großen Einstellbereich bilden, der mittendrin keine "Löcher", also Bereiche, auf die nicht eingestellt werden kann, aufweisen darf.

Es ist von Vorteil, dass das Verschlussmittel ein Magnetschloss ist, das ein Gehäuse mit einem oder mehreren zwischen einer Verriegelungsposition und einer Öffnungsposition beweglichen Regelelementen und mit einer Steckverbindung zwischen dem Gehäuse und dem Steckschaft eines Sockelteils aufweist, wobei das Gehäuse eine Einstecköffnung aufweist und der Steckschaft mit einer Sperrrille versehen ist, in welche die Riegelelemente eingreifen können, um das Sockelteil im Gehäuse zu verriegeln, wobei das Sockelteil im Gehäuse mittels eines Schlüssels, insbesondere eines Magnetschlüssels, wieder entriegelbar ist.

Vorteilhafterweise sind die Schlaufen als Metallschlaufen ausgebildet.

Vorzugsweise zweiteilt die Schlaufe den zweiten Befestigungsgurt. Es hat sich herausgestellt, dass durch diese Anordnung die Zahl der Ösen von 46 auf 12 reduziert werden kann. Auch reduziert sich die Gesamtlänge (Schnittmaße) eines Befestigungsgurtes von ca. 1,5 m auf nur ca. 1,37 m, d.h. um ca. 25%.

Dazu dient auch, dass sich in dem ersten Befestigungsgurt in der nicht zum Haltegurt zugewendeten Hälfte 9 Ösen im Abstand von ca. 2 cm und in der im Haltegurt zugewandten Hälfte des ersten Befestigungsgurtes 3 Ösen befinden, wobei der Abstand vom Haltegurt bis zur ersten Öse in der nicht zum Haltegurt zugewendeten Hälfte ca. 51 cm und der Abstand vom Haltegurt zur dritten Öse in der von Haltegurt zugewandten Hälfte ca. 29 cm beträgt sowie der Abstand zum Haltegurt zur ersten Öse in der zum Haltegurt zugewandten Hälfte ca. 11 cm und der Abstand zwischen den Ösen in der zum Haltegurt zugewandten Hälfte ca. 9 cm erreicht. Auch sollte der Abstand zwischen der Schlaufe, die den zweiten Befestigungsgurt zweiteilt, und dem Haltegurt ca. 10 cm betragen.

Eine weitere praktikable Variante, einen möglichst großen Einstellbereich zu bekommen, besteht darin, dass sich an dem Haltegurt Befestigungsgurte anschließen, die eine unterschiedliche Länge und jeweils eine Schlaufe aufweisen.

Es kann sich dabei als Vorteil erweisen, dass derartige Befestigungsgurte weitere Schlaufen aufweisen.

### Kurze Beschreibung der Zeichnungen

Im Folgenden wird die Erfindung mit Hilfe der Figuren beispielhaft erläutert. Es zeigen in schematischen Darstellungen:
- Fig. 1: eine schaubildliche Ansicht eines erfindungsgemäßen Gurtes,
- Fig. 2: einen Schnitt durch ein Magnetschloss,
- Fig. 3: eine schaubildliche Ansicht einer erfindungsgemäßen Halterung einer Leibbandage,
- Fig. 4: eine schaubildliche Ansicht einer erfindungsgemäßen Halterung einer Leibbandage, bei der Befestigungsgurte an einem Haltegurt befestigt sind und
- Fig. 5: eine schaubildliche Ansicht einer Halterung einer Leibbandage, bei der Befestigungsgurte an einem Haltegurt befestigt sind.

### Bester Weg zur Ausführung der Erfindung

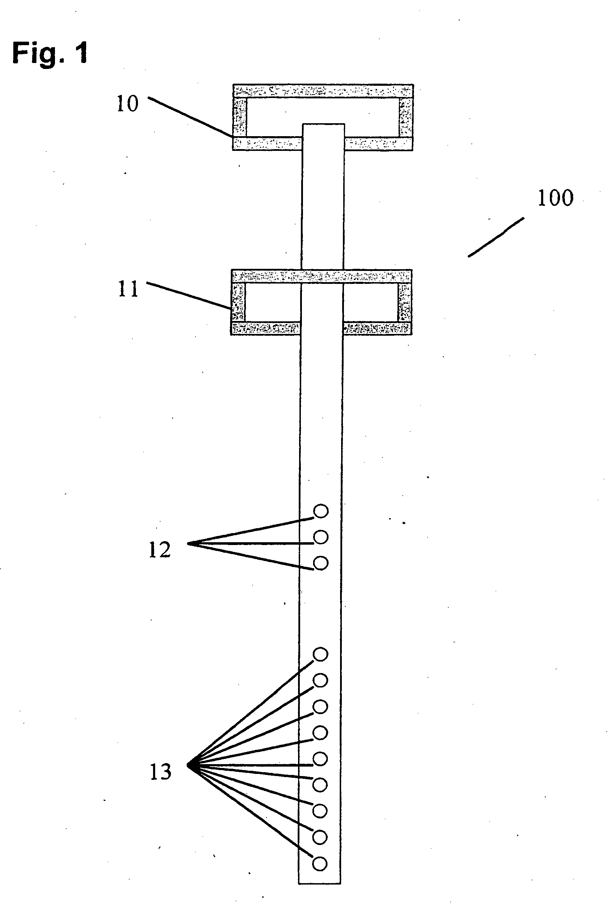

Fig. 1 zeigt einen Gurt 100, der mehrere Schlaufen 10, 11 aufweist. Eine Schlaufe 10 kann dabei an einem Ende des Gurtes 100 angeordnet sein. Der Gurt 100 weist zudem Ösen 12 für die Aufnahme des Verschlussmittels auf. Zwischen der Schlaufe 10, die an einem Ende des Gurtes 100 angeordnet ist, und den Ösen 12 befindet sich eine weitere Schlaufe 11. Beide Schlaufen 10 und 11 können dabei als Metallschlaufen ausgebildet sein. Zudem befinden sich an dem Ende des Gurtes 100, an dem nicht die Schlaufe 10 angeordnet ist, weitere Ösen 13. In der räumlichen Reihenfolge der Ösen 12, 13 sind die für das Verschlussmittel vorgesehenen Ösen 12 der Schlaufe 11 am nächsten. Zum Verschließen des Gurtes 100 kann der Gurt 100 durch die Schlaufe 11 gezogen sowie umgeschlagen werden, so dass die Ösen 12, 13 des Gurtes 100 aufeinander liegen. Dadurch, dass nun mehrere Ösen 12 für die Aufnahme des Verschlussmittels vorgesehen sind und der Gurt 100 eine weitere Schlaufe 11 aufweist, ergeben sich mehrere Einstellbereiche. Beispielsweise ergeben sich sechs Einstellbereiche, wenn der Gurt 100 mit zwei Schlaufen 10, 11 versehen ist und drei Ösen 12 in dem Gurt 100 für die Aufnahme des Verschlussmittels vorgesehen sind. Weitere Einstellbereiche lassen sich realisieren, wenn der Gurt weitere Schlaufen aufweist und das Verschlussmittel für weitere Ösen vorgesehen ist.

Auf diese Weise wird gewissermaßen das Prinzip einer Gangschaltung realisiert. Die Zahl der möglichen Einstellbereiche bei dem erfindungsgemäßen Gurt 100 wird also durch das Hinzufügen von Schlaufen in dem Gurt 100 und durch Vergrößerung der Anzahl der Ösen 12, die für die Aufnahme des Verschlussmittels vorgesehen sind, erhöht. Es wird folglich eine Vielzahl von kleinen Einstellbereichen geschaffen, die einander anschließen und auf diese Weise den gewünschten einheitlichen großen Einstellbereich bilden.

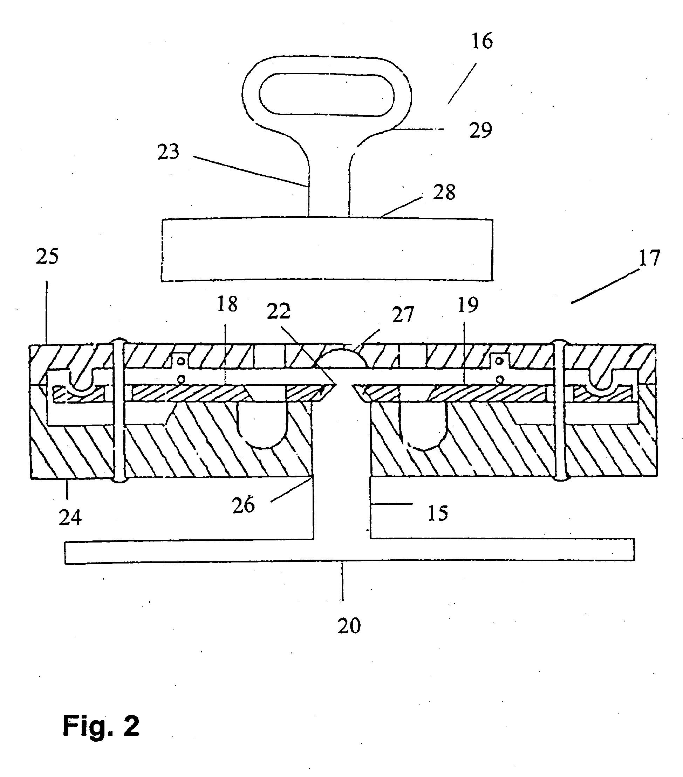

Fig. 2 zeigt einen Schnitt durch ein Magnetschlossgehäuse 17 eines Magnetschlosses 16 als Verschlussmittel, welches aus der Unterschale 24 und der Oberschale 25 zusammengesetzt ist. Zusammen mit dem Schlüssel 23 und dem Sockelteil 20 bilden sie das, vorbekannte Magnetschloss 16 aus der DE-PS 1 942 535. Die Riegelelemente 18, 19 sind als Sperrscheiben ausgebildet und kippbar angeordnet. Sie sind dabei derart gestaltet, dass sie in der Verriegelungsposition teilweise in den Bereich der Einstecköffnung 26 hineinragen. Wenn der Steckschaft 15 in die Einstecköffnung 26 eingesteckt wird, drückt der Sockelkopf die federbelasteten Riegelelemente 18, 19 zunächst zurück. Sobald der Steckschaft 15 in die Einstecköffnung 26 ganz eingeführt ist, greifen die Riegelelemente 18, 19 in der Verriegelungsposition in die Sperrrille 22 des Sockelteils 20 ein. Ein Zurückziehen des Sockelteils 20 aus dem Magnetschlossgehäuse 17 ist daher verhindert, da der Sockelkopf 27 auf den Riegelelementen 18, 19 aufsitzt. Auf diese Weise ist eine Verankerung zwischen Magnetschlossgehäuse 17 und Sockelteil 20 gegeben. Ein Herausziehen des Steckschaftes 15 ist erst dann möglich, wenn die Riegelelemente 18, 19 durch eine seitliche Verschiebung soweit zur Seite bewegt worden sind, dass sie die Sperrrille 22 verlassen können. Das Zurückweichen der Riegelelemente 18, 19 aus der Sperrrille 22 kann beispielsweise durch den Schlüssel 23 mit einer Magnetplatte 28 und mit einem Griff 29 bewirkt werden. Durch Anlegen des Schlüssels 23 an den Boden des Magnetschlossgehäuses 17 wird eine Anzugskraft auf die aus Stahl oder anderem magnetischen Material bestehenden Regelelemente 18, 19 ausgeübt und die Regelelemente 18, 19 dadurch entgegen einer axial wirkenden Rückstellfeder zum Kicken gebracht werden. Der Teil des Steckschaftes 15, der zum Zwecke einer Steckverbindung zwischen Sockelteil 20 und Magnetschlossgehäuse 17 in der Einstecköffnung 21 steckt, sowie die zum Querschnitt des Sockelschaftes 15 korrespondierende Einstecköffnung 26 sind dabei sechseckig ausgebildet. Durch die sechseckige Ausbildung der Querschnitte von Steckschaft 15 und Einstecköffnung 26 ist eine Verdrehsicherung zwischen dem Magnetschlossgehäuse 17 und dem Sockelteil 20 gegeben.

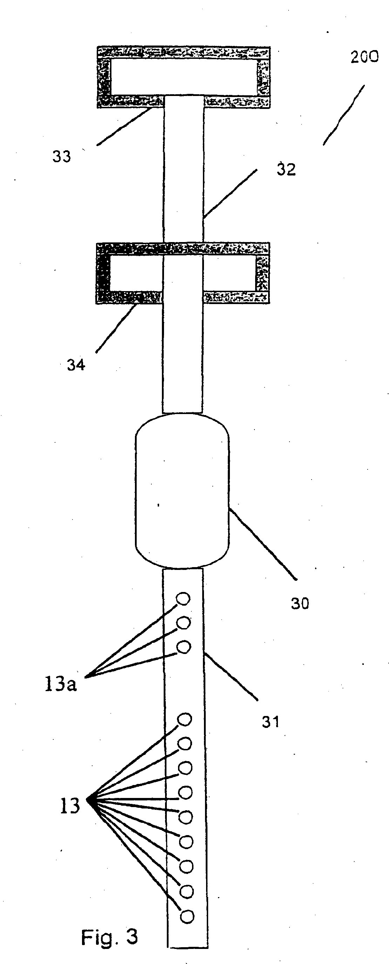

Die große Anzahl von Ösen 13, 13a kann auch durch die in Fig. 3 gezeigte Halterung einer Leibbandage 200 erheblich reduziert und so der Gurt selbst verkürzt werden. Die Halterung weist einen Haltegurt 30 sowie einen ersten Befestigungsgurt 31 und einen zweiten Befestigungsgurt 32 auf. Auch kann bei der Halterung am Ende des zweiten Befestigungsgurtes 32 eine Schlaufen 33 angeordnet sein, die auch als Metallschlaufe ausgebildet sein kann. Der zweite Befestigungsgurt 32 weist jedoch mehrere Schlaufen auf. Beispielsweise kann der Befestigungsgurt 32 die weitere Schlaufe 34 aufweisen, die nicht am Gurtende angeordnet ist und den zweiten Befestigungsgurt 32 zweiteilt. Zum Erschließen der Halterung wird der erste Befestigungsgurt 31 nun durch die Schlaufe 34 gezogen sowie umgeschlagen, so dass die Ösen 13, 13a des ersten Befestigungsgurtes 31 aufeinander liegen. Dadurch, dass nun mehrere Ösen 13a für die Schlossaufnahme vorgesehen sind und sich in dem zweiten Befestigungsgurt eine zusätzliche Schlaufe befindet, ergeben sich mehrere Einstellbereiche. In der räumlichen Reihenfolge der Ösen 13, 13a sind die für das Verschlussmittel vorgesehenen Ösen 13a direkt neben dem Haltegurt 30 angeordnet. Beispielsweise ergeben sich sechs Einstellbereiche, wenn der zweite Befestigungsgurt 32 mit zwei Schlaufen 33, 34 versehen ist und drei Ösen 13a in dem ersten Befestigungsgurt 31 für die Schlossaufnahme vorgesehen sind. Auf diese Weise wird gewissermaßen das Prinzip einer Gangschaltung realisiert. Die Zahl der möglichen Einstellbereiche bei der erfindungsgemäßen Leibbandage wird also durch das Hinzufügen von Schlaufen in dem zweiten Befestigungsgurt 32 und durch die Vergrößerung der Anzahl der Ösen 13a, die für die Schlossaufnahme vorgesehen sind, erhöht. Es wird folglich eine Vielzahl von kleinen Einstellbereichen geschaffen, die einander anschließen und auf diese Weise den gewünschten einheitlichen großen Einstellbereich bilden.

In dem ersten Befestigungsgurt 31 in der nicht zum Haltegurt 30 zugewendeten Hälfte befinden sich neun Ösen 13 im Abstand von ca. 2 cm. In der im Haltegurt 30 zugewandten Hälfte des ersten Befestigungsgurtes 31 befinden sich dagegen drei Ösen 13a, wobei der Abstand vom Haltegurt 30 bis zur ersten Öse 13 der nicht zum Haltegurt 30 zugewandten Hälfte ca. 51 cm und der Abstand vom Haltegurt 30 zur dritten Öse 13a in der zum Haltegurt 30 zugewandten Hälfte ca. 29 cm beträgt. Der Abstand vom Haltegurt 30 zur ersten Öse 13a in der zum Haltegurt 30 zugewandten Hälfte beträgt dagegen ca. 11 cm und der Abstand zwischen den Ösen 13a in der zum Haltegurt 30 zugewandten Hälfte weist eine Länge von ca. 9 cm auf. Weiterhin beträgt der Abstand zwischen der Schlaufe 34 und dem Haltegurt 30 ca.10 cm. Der zweite Befestigungsgurt 32 weist vorzugsweise eine Länge von ca. 35 cm auf, wohingegen der erste Befestigungsgurt 31 eine Länge von ca. 67 cm hat.

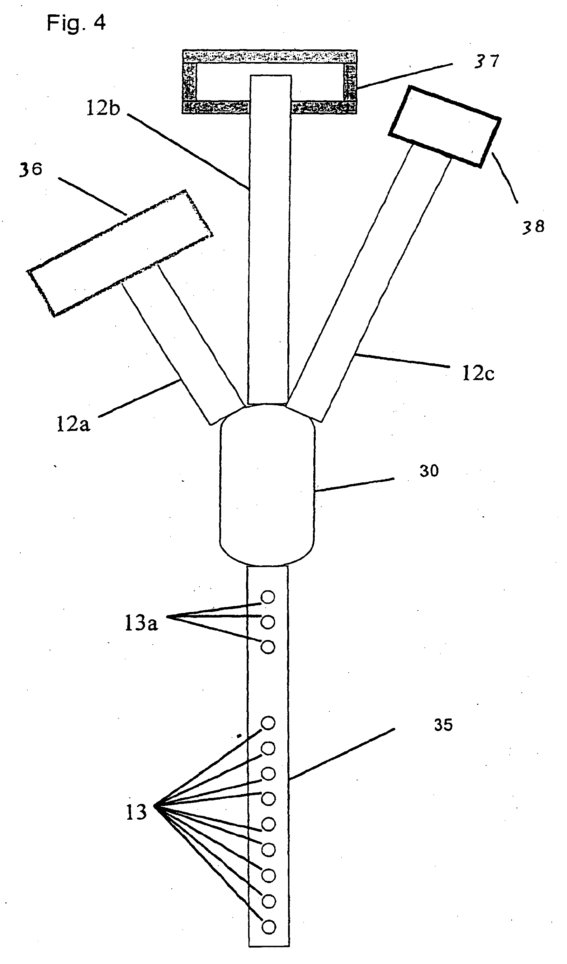

Aus Fig. 4 geht noch einmal eine praktikable Variante hervor, einen möglichst großen Einstellbereich zu bekommen, in dem sich an dem Haltegurt 30 Befestigungsgurte 12a, 12b, 12c anschließen, die eine unterschiedliche Länge und jeweils eine Schlaufe aufweisen. Zum Verschließen der Befestigungsgurte 35, 12a, 12b, 12c wird der erste Befestigungsgurt 35 durch eine der Schlaufen 36, 37, 38 gezogen, so dass eine Zahl der Ösen 13, 13a aufeinander liegen. Auch bei dieser Variante lässt sich also das Prinzip einer Gangschaltung realisieren.

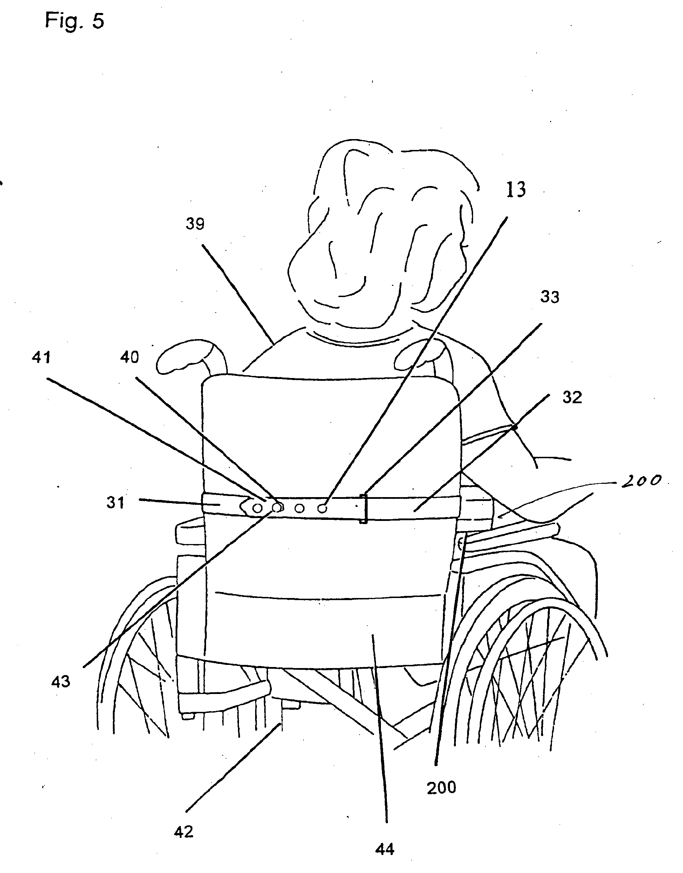

Fig. 5 zeigt eine Halterung einer Leibbandage, 200 zur Begrenzung der Beweglichkeit einer in einer Sitzgelegenheit 42 befindlichen Person 39, wobei es sich bei der Sitzgelegenheit 42 um einen Rollstuhl handelt. Die Halterung weist einen Haltegurt (nicht dargestellt) auf, an dem sich ein erster Befestigungsgurt 31 und der dem ersten Befestigungsgurt 31 gegenüberliegender zweiter Befestigungsgurt 32 anschließt, wobei die beiden Befestigungsgurte 31, 32 um die Rückenlehne 44 des Rollstuhls legbar sowie erfindungsgemäß miteinander verbunden sind. Der erste Befestigungsgurt 31 ist mit einem Verschlussmittel 41 versehen, welches als Magnetschloss 40 mit einem Gehäuse 43 ausgebildet ist.

### Bezugszeichenliste

- 200: Leibbandage
- 100: Gurt
- 10: Schlaufen
- 11: Schlaufen
- 12: Ösen
- 12a, b, c: Befestigungsgurte
- 13: Ösen
- 13a: Ösen für die Aufnahme des Schlosses
- 14: Verschlussmittel
- 15: Steckschaft
- 16: Magnetschloss
- 17: Magnetschlossgehäuse
- 18: Riegelelement
- 19: Riegelelement
- 20: Sockelteil
- 23: Schlüssel
- 24: Unterschale
- 25: Oberschale
- 26: Einstecköffnung
- 27: Sockelkopf
- 28: Magnetplatte
- 29: Griff
- 30: Haltegurt
- 31: erster Befestigungsgurt
- 32: zweiter Befestigungsgurt
- 33: Schlaufe
- 34: Schlaufe
- 35: Befestigungsgurt
- 36: Schlaufe
- 37: Schlaufe
- 38: Schlaufe
- 39: Person
- 40: Magnetschloss
- 41: Verschlussmittel
- 42: Sitzgelegenheit
- 43: Gehäuse
- 44: Rückenlehne

## Patentansprüche

1. Gurt (100), vorzugsweise aus Textilware oder Lederware, mit Ösen (12, 13), **dadurch gekennzeichnet, dass** an den Ösen (12) ein Verschlussmittel anbringbar ist, welches den Gurt (100) verschließen kann, und der Gurt (100) Schlaufen (10, 11) aufweist, wobei zum Verschließen des Gurtes (100) der Gurt (100) durch eine der Schlaufen (10, 11) gezogen ist und eine Zahl der Ösen (12, 13) aufeinanderliegen und der Gurt (100) mit einem Verschlussmittel (14) versehen ist, das einen Steckschaft (15) aufweist, der durch die Ösen (12, 13) hindurchführbar ist.

2. Gurt nach Anspruch 1, **dadurch gekennzeichnet, dass** der Gurt (100) durch mindestens zwei Schlaufen (10, 11) gleichzeitig durchführbar ist.

3. Gurt nach Anspruch 1 oder 2, **dadurch gekennzeichnet, dass** der Gurt (100) zwei Schlaufen (10, 11) aufweist und das Verschlussmittel (14) für drei Ösen (12) vorgesehen ist.

4. Gurt nach Anspruch 1 oder 2, **dadurch gekennzeichnet, dass** der Gurt (100) drei Schlaufen (10, 11) aufweist und das Verschlussmittel (14) für vier Ösen (12) vorgesehen ist.

5. Gurt nach einem der Ansprüche 1 bis 4, **dadurch gekennzeichnet, dass** das Verschlussmittel (14) ein Magnetschloss (15) mit einem Gehäuse (17) mit einem oder mehreren zwischen einer Verriegelungsposition und einer Öffnungsposition beweglichen Riegelelementen (18, 19) und mit einer Steckverbindung zwischen dem Gehäuse (17) und dem Steckschaft (15) eines Sockelteils (20) ist, wobei das Gehäuse (17) eine Einstecköffnung (21) aufweist und der Steckschaft (15) mit einer Sperrrille (22) versehen ist, in welche die Riegelelemente (18, 19) eingreifen können, um das Sockelteil (20) im Gehäuse (17) zu verriegeln, wobei das Sockelteil (20) im Gehäuse (17) mittels eines Schlüssels (23), insbesondere eines Magnetschlüssels (23), wieder entriegelbar ist.

6. Gurt einem der vorhergehenden Ansprüche, **dadurch gekennzeichnet, dass** die Schlaufen (10, 11) als Metallschlaufen ausgebildet sind.

7. Verwendung eines Gurtes gemäß einem der Ansprüche 1 bis 6 für eine Leibbandage (200) zur Fixierung und/oder Sicherung gegen Herausfallen einer in einer Sitzgelegenheit (42), insbesondere in einem Rollstuhl oder auf einer Trage, befindlichen Person (39), die einen Haltegurt (30) aufweist, an dem sich ein erster Befestigungsgurt (31) und ein dem ersten Befestigungsgurt (31) gegenüberliegender zweiter Befestigungsgurt (32) anschließen, wobei die beiden Befestigungsgurte (31, 32) um eine Rückenlehne (44) der Sitzgelegenheit (42) legbar sowie miteinander verschließbar sind und der erste Befestigungsgurt (31) Ösen (13, 13a) aufweist, **dadurch gekennzeichnet, dass** an den Ösen (13a) ein Verschlussmittel (41) anbringbar ist, welches die Befestigungsgurte (31, 32) miteinander verschließen kann, und der zweite Befestigungsgurt (32) Schlaufen (33, 34) aufweist, wobei zum Verschließen der Befestigungsgurte (31, 32) der erste Befestigungsgurt (31) durch eine der Schlaufen (33, 34) gezogen ist und eine Zahl der Ösen (13, 13a) aufeinanderliegen und der erste Befestigungsgurt (31) mit dem Verschlussmittel (41) versehen ist.

## Claims

1. Belt (100), preferably made of textiles or leather, having eyelets (12, 13) **characterised in that** a closure means which can close the belt (100) can be attached to the eyelets (12), and the belt (100) has loops (10, 11), wherein for the purpose of closing the belt (100), the belt (100) is pulled through one of the loops (10, 11) and a number of the eyelets (12, 13) lie one above the other and the belt (100) is provided with a closure means (14), which has a plug-in shaft (15) which can be guided through the eyelets (12, 13).

2. Belt according to claim 1, **characterised in that** the belt (100) is guideable simultaneously through at least two loops (10, 11).

3. Belt according to claim 1 or 2, **characterised in that** the belt (100) has two loops (10, 11) and the closure means (14) is provided for three eyelets (12).

4. Belt according to claim 1 or 2, **characterised in that** the belt (100) has three loops (10, 11) and the closure means (14) is provided for four eyelets (12).

5. Belt according to any one of claims 1 to 4, **characterised in that** the closure means (14) is a magnetic lock (15) comprising a casing (17) having one or more locking elements (18, 19) movable between a locking position and an opening position and having a plug-in connection between the casing (17) and the plug-in shaft (15) of a base part (20), wherein the casing (17) has a plug-in opening (21) and the plug-in shaft (15) is provided with a locking groove (22) into which the locking elements (18, 19) can engage in order to lock the base part (20) in the casing (17), wherein the base part (20) in the casing (17) is unlockable again by means of a key (23), in particular a magnetic key (23).

6. Belt according to any of the preceding claims, **characterised in that** the loops (10, 11) are configured as metal loops.

7. Use of a belt according to any one of claims 1 to 6 for a body bandage (200) for fixing and/or securing against falling out a person (39) located in a seat (42), in particular in a wheelchair or on a stretcher, which has a retaining belt (30) which is followed by a first fastening belt (31) and a second fastening belt (32) opposite the first fastening belt (31), wherein the two fastening belts (31, 32) can be placed around a back rest (44) of the seat (42) and can be closed with one another and the first fastening belt (31) has eyelets (13, 13a), **characterised in that** a closure means (41) is attachable to the eyelets (13a) which can close the fastening belts (31, 32) with one another and the second fastening belt (32) has loops (33, 34) wherein for closing the fastening belts (31, 32) the first fastening belt (31) is pulled through one of the loops (33, 34) and a number of the eyelets (13, 13a) lie one upon the other and the first fastening belt (31) is provided with the closure means (41).

## Revendications

1. Ceinture (100), de préférence en textile ou en cuir, comportant des oeillets (12, 13), **caractérisée en ce qu'**on peut poser au niveau des billets (12) un moyen de fermeture qui peut fermer la ceinture (100) et que la ceinture (100) présente des boucles (10, 11), sachant que, pour fermer la ceinture (100), la ceinture (100) est tirée dans un des boucles (10, 11) et qu'un certain nombre d'oeillets (12, 13) sont superposés et que la ceinture (100) est pourvue d'un moyen de fermeture (14) qui présente une tige enfichable (15) qui peut être passée dans les billets (12, 13).

2. Ceinture selon la revendication 1, **caractérisée en ce que** la ceinture (100) peut être passée en même temps dans au moins deux boucles (10, 11).

3. Ceinture selon la revendication 1 ou 2, **caractérisée en ce que** la ceinture (100) présente deux boucles (10, 11) et que le moyen de fermeture (14) est prévu pour trois billets (12).

4. Ceinture selon la revendication 1 ou 2, **caractérisée en ce que** la ceinture (100) présente trois boucles (10, 11) et que le moyen de fermeture (14) est prévu pour quatre oeillets (12).

5. Ceinture selon une des revendications 1 à 4, **caractérisée en ce que** le moyen de fermeture (14) est une serrure magnétique (15) dotée d'un boîtier (17) comportant un ou plusieurs éléments de verrou (18, 19) mobiles entre une position de verrouillage et une position d'ouverture et un raccord fiché entre le boîtier (17) et la tige enfichable (15) d'une partie socle (20), le boîtier (17) présentant un orifice de fichage (21) et la tige enfichable (15) étant pourvue d'une rainure d'arrêt (22) dans laquelle les éléments de verrou (18, 19) peuvent s'engrener pour verrouiller la partie socle (20) dans le boîtier (17), la partie socle (20) étant déverrouillable dans le boîtier (17) au moyen d'une clé (23), en particulier d'une clé magnétique (23) .

6. Ceinture selon une des revendications précédentes, **caractérisée en ce que** les boucles (10, 11) se présentent sous forme de boucles métalliques.

7. Utilisation d'une ceinture selon une des revendications 1 à 6 comme bandage corporel (200) pour fixer et/ou sécuriser contre toute chute une personne (39) assise dans un siège (42), notamment dans un fauteuil roulant ou sur une civière, présentant une ceinture de retenue (30) à laquelle se raccordent une première ceinture de fixation (31) et une seconde ceinture de fixation (32) opposée à la première ceinture de fixation (31), les deux ceintures de fixation (31, 32) pouvant être posées autour d'un dossier (44) du siège (42) et être fermées ensemble et la première ceinture de fixation (31) comportant des billets (13, 13a), **caractérisée en ce que** peut être installé sur les billets (13a) un moyen de fermeture (41) qui peut fermer les ceintures de fixation (31, 32) ensemble et que la seconde ceinture de fixation (32) présente des boucles (33, 34), sachant que, pour fermer les ceintures de fixation (31, 32), la première ceinture de fixation (31) est tirée dans une des boucles (33, 34) et qu'un certain nombre d'oeillets (13, 13a) sont superposés et que la première ceinture de fixation (31) est équipée du moyen de fermeture (41).
